(19) Europäisches Patentamt
European Patent Office
Office européen des brevets

(11) EP 4 578 839 A1

(12) EUROPEAN PATENT APPLICATION

(43) Date of publication:
**02.07.2025 Bulletin 2025/27**

(21) Application number: **24217147.8**

(22) Date of filing: **03.12.2024**

(51) International Patent Classification (IPC):
***C03C 17/00*** *(2006.01)* ***C03C 17/32*** *(2006.01)*

(52) Cooperative Patent Classification (CPC):
**C03C 17/32;** C03C 2217/75

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.12.2023 JP 2023219343**

(71) Applicant: **Sumitomo Rubber Industries, Ltd.**
**Kobe-shi, Hyogo 651-0072 (JP)**

(72) Inventor: **MINAGAWA, Yasuhisa**
**Kobe-shi, 651-0072 (JP)**

(74) Representative: **Manitz Finsterwald**
**Patent- und Rechtsanwaltspartnerschaft mbB**
**Martin-Greif-Strasse 1**
**80336 München (DE)**

(54) **POLYMER-COATED GLASS SUBSTRATE**

(57) Provided is a polymer-coated glass substrate having a surface with controlled irregularities and a low elastic modulus. The polymer-coated glass substrate includes a glass substrate and a hydrophilic polymer layer including a blend of hydrophilic polymers with different molecular weights, the hydrophilic polymer layer being on a surface of the glass substrate.

EP 4 578 839 A1

## Description

### TECHNICAL FIELD

The present invention relates to a polymer-coated glass substrate.

### BACKGROUND ART

A technique of coating the surface of a glass substrate with a special polymer has been proposed to produce a device for adsorbing specific cells (e.g., blood cells and cells in blood or biological fluid such as cancer cells, stem cells, and T-cells) from blood or biological fluid.

However, coating with some special polymers is difficult to form a flat smooth surface. Since irregularities of the surface affect the adsorption of specific cells, there has been a request for a substrate which has a surface with controlled irregularities to be excellent in, for example, adsorbing specific cells such as cancer cells, stem cells, and T-cells (see, Patent Literature 1). Moreover, large surface irregularities are likely to cause white turbidity of the coating surface. Therefore, the irregularities of the surface are desirably controlled to suppress white turbidity of the coating surface. Suppression of white turbidity of the coating surface is expected to improve adsorption, etc. of specific cells such as cancer cells, stem cells, and T-cells.

### CITATION LIST

### PATENT LITERATURE

Patent Literature 1: JP 2005-523981 T

### SUMMARY OF INVENTION

### TECHNICAL PROBLEM

The present invention aims to solve the above problem and provide a polymer-coated glass substrate having a surface with controlled irregularities and a low elastic modulus.

### SOLUTION TO PROBLEM

The present invention relates to a polymer-coated glass substrate including a glass substrate and a hydrophilic polymer layer including a blend of hydrophilic polymers with different molecular weights, the hydrophilic polymer layer being on a surface of the glass substrate.

### ADVANTAGEOUS EFFECTS OF INVENTION

The polymer-coated glass substrate according to the present invention includes a glass substrate and a hydrophilic polymer layer including a blend of hydrophilic polymers with different molecular weights, the hydrophilic polymer layer being on a surface of the glass substrate. Thus, the present invention can provide a polymer-coated glass substrate having a surface with controlled irregularities and a low elastic modulus. Such a polymer-coated glass substrate exhibits an effect of improving adsorption of specific cells such as cancer cells, stem cells, and T-cells.

### DESCRIPTION OF EMBODIMENTS

The polymer-coated glass substrate includes a glass substrate and a hydrophilic polymer layer including a blend of hydrophilic polymers with different molecular weights. The hydrophilic polymer layer is on a surface of the glass substrate. The irregularities of the substrate surface can be controlled by the hydrophilic polymer layer formed using a blend of hydrophilic polymers with different molecular weights on the surface of the glass substrate, whereby it is possible to provide a polymer-coated glass substrate coated with a hydrophilic polymer layer having a low surface roughness, a high flat smoothness, and a low surface elastic modulus.

The number of tumor cells (cancer cells, etc.) appearing in biological fluid, such as circulating tumor cells (several to hundreds of cells/1 ml of blood), is very small, and it is considered important to adsorb tumor cells present in the sampled biological fluid as many as possible to analyze them. The polymer-coated glass substrate includes a hydrophilic polymer layer including a blend of hydrophilic polymers with different molecular weights on the glass substrate. The hydrophilic

polymer layer has a surface with controlled irregularities, a low roughness, and a high flat smoothness is formed. The surface of the formed hydrophilic polymer layer has a low elastic modulus. The irregularities of the hydrophilic polymer layer affect the adsorption of specific cells such as cancer cells, stem cells, and T-cells. The surface with controlled irregularities and a high flat smoothness is excellent in adsorbing specific cells. The elastic modulus of the surface affects the adsorption of specific cells. The lower the elastic modulus is, the more the adsorption is improved. Yet, a surface with an excessively low elastic modulus has a low adsorption capacity. In other words, the elastic modulus has an optimal value. For example, the polymer-coated glass substrate including a blend of hydrophilic polymers with different molecular weights on the surface of the glass substrate is usable to determine the number of tumor cells in biological fluid by adsorbing tumor cells and counting the number of adsorbed tumor cells, thereby evaluating the cancer-treating effect. Moreover, the adsorbed tumor cells may be cultured and then used to determine the effects of anticancer drugs and the like, so that the effects of drugs such as anticancer drugs can be confirmed ex vivo before administration. This technique is also usable to screen drugs such as anticancer drugs. Additionally, genetic analysis may be performed on the adsorbed or cultured tumor cells to examine the mutation and expression of the tumor cells. The analysis result may be used to screen drugs such as anticancer cells or may be utilized to elucidate the mechanisms of cancers.

The type of glass in the polymer-coated glass substrate is not limited. Examples include soda lime glass, alkali-free glass, borosilicate glass ($SiO_2$-$B_2O_3$-ZnO glass, $SiO_2$-$B_2O_3$-$Bi_2O_3$ glass, etc.), potash glass, crystal glass (glass containing PbO, such as $SiO_2$-PbO glass, $SiO_2$-PbO-$B_2O_3$ glass, and $SiO_2$-$B_2O_3$-PbO glass), titanium crystal glass, barium glass, boron glass ($B_2O_3$-ZnO-PbO glass, $B_2O_3$-ZnO-$Bi_2O_3$ glass, $B_2O_3$-$Bi_2O_3$ glass, $B_2O_3$-ZnO glass, etc.), strontium glass, alumina silicate glass, soda-zinc glass, and soda-barium glass (BaO-$SiO_2$ glass, etc.). These types of glass may be used alone or in combinations of two or more.

The contact angle of an air bubble on the glass substrate in water is preferably 100° or more and 120° or less. The contact angle is more preferably 103° or higher and more preferably 117° or lower.

Herein, the term "contact angle of an air bubble in water" refers to a contact angle measured by putting an air bubble (air) onto the substrate surface in water and measuring a contact angle between the air bubble and the substrate surface.

Although the thickness of the glass substrate is not limited, the average thickness is preferably 100 um or more and 4000 um or less, more preferably 100 um or more and 3000 um or less. The term "average thickness" refers to an average of the thicknesses measured at ten arbitrary points using a micrometer.

The polymer-coated glass substrate includes a hydrophilic polymer layer including a blend of hydrophilic polymers with different molecular weights (hereinafter, also referred to as "hydrophilic polymer blend layer") on the surface of the glass substrate. The hydrophilic polymers with different molecular weights may include two polymers of the same type with different molecular weights (for example, the blend contains hydrophilic polymers including poly(2-methoxyetyl acrylate) having a number average molecular weight of 16000 and poly(2-methoxyetyl acrylate) having a number average molecular weight of 80000) or may include different hydrophilic polymers (hydrophilic polymers having different structures) with different molecular weights (for example, the blend contains hydrophilic polymers including poly(2-methoxyetyl acrylate) having a number average molecular weight of 16000 and polyacryloylmorpholine having a number average molecular weight of 80000).

To better achieve the advantageous effect, the hydrophilic polymer blend layer desirably includes hydrophilic polymers of the same type with different molecular weights.

The hydrophilic polymers in the hydrophilic polymer blend layer are not limited and may be appropriate known hydrophilic polymers, for example. A type of hydrophilic polymer may be used alone or in combinations of two or more types.

Each hydrophilic polymer can be produced by known methods. For example, it may be synthesized by polymerizing a hydrophilic monomer of the target hydrophilic polymer by a known method using a solution of the hydrophilic monomer. Non-limiting examples of the solvent of the hydrophilic monomer solution include those described later. Toluene or methanol is preferred among these.

Examples of the hydrophilic polymers include homopolymers or copolymers of one or two or more hydrophilic monomers, and copolymers of one or two or more hydrophilic monomers and one or two or more different monomers. The polymer-coated glass substrate includes the hydrophilic polymer blend layer including a blend of such hydrophilic polymers with different molecular weights.

Non-limiting examples of the hydrophilic monomer include various monomers containing hydrophilic groups. Examples of the hydrophilic groups include known hydrophilic groups such as an amide group, a sulfuric acid group, a sulfonic acid group, a carboxylic acid group, a hydroxy group, an amino group, and an oxyethylene group.

Specific examples of the hydrophilic monomer include (meth)acrylic acids; (meth)acrylic acid esters; alkoxyalkyl (meth)acrylates such as methoxyethyl (meth)acrylates; hydroxyalkyl (meth)acrylates such as hydroxyethyl (meth) acrylates; (meth)acrylamides; and (meth)acrylamide derivatives containing cyclic groups such as (meth)acryloylmorpholines. Of these, (meth)acrylic acids, (meth)acrylic acid esters, alkoxyalkyl (meth)acrylates, and (meth)acrylamide derivatives containing cyclic groups are preferred; alkoxyalkyl (meth)acrylates and (meth)acryloylmorpholines are more

preferred; alkoxyalkyl (meth)acrylates are still more preferred; and 2-methoxyethylacrylate is particularly preferred. Each of these may be used alone or in combinations of two or more.

The different monomer may be appropriately selected from those which do not inhibit the effect of the hydrophilic polymer. Specific examples of the different monomer include aromatic monomers such as styrene, vinyl acetate, and N-isopropylacrylamide which can impart temperature responsiveness. Each of these may be used alone or in combinations of two or more.

Specific examples of the homopolymers and copolymers include homopolymers formed from a single hydrophilic monomer, such as polyacrylic acids, polyacrylic acid esters, polymethacrylic acids, polymethacrylic acid esters, polyacryloylmorpholines, polymethacryloylmorpholines, polyacrylamides, polymethacrylamides, polyalkoxyalkyl acrylates, and polyalkoxyalkyl methacrylates; copolymers formed from two or more hydrophilic monomers listed above; and copolymers formed from one or more hydrophilic monomers listed above and one or more different monomers listed above.

The hydrophilic polymers in the hydrophilic polymer blend layer preferably include hydrophilic polymers with different molecular weights represented by the formula (I) below. The hydrophilic polymers with different molecular weights represented by the formula (I) may include two or more hydrophilic polymers which include the same structural unit and have different molecular weights (for example, the hydrophilic polymers include poly(2-methoxyetyl acrylate) having a number average molecular weight of 16000 and poly(2-methoxyetyl acrylate) having a number average molecular weight of 80000) or may include two or more hydrophilic polymers which include different structural units and have different molecular weights (for example, the hydrophilic polymers include poly(2-methoxyetyl acrylate) having a number average molecular weight of 16000 and poly(2-ethoxyetyl methacrylate) having a number average molecular weight of 80000). One type or two or more types of the hydrophilic polymers represented by the formula (I) may be used.

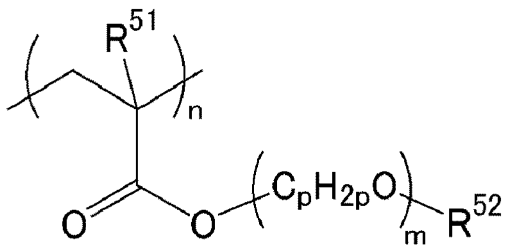

(I)

In the formula, $R^{51}$ represents a hydrogen atom or a methyl group; $R^{52}$ represents an alkyl group; p represents 1 to 8; m represents 1 to 5; and n represents the number of repetitions.

Suitable examples of the hydrophilic polymers represented by the formula (I) include hydrophilic polymers represented by the following formula (I-1):

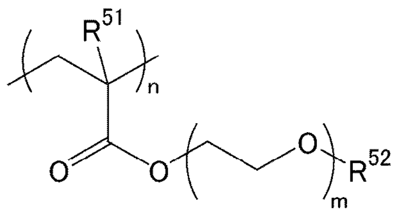

(I-1)

wherein $R^{51}$ represents a hydrogen atom or a methyl group; $R^{52}$ represents an alkyl group; m represents 1 to 5; and n represents the number of repetitions. Each of the hydrophilic polymers represented by the formula (I-1) may be used alone or in combinations of two or more.

The hydrophilic polymers may suitably include a copolymer of hydrophilic monomers with different molecular weights represented by the formula (II) below and a different monomer.

The copolymer of hydrophilic monomers with different molecular weights represented by the formula (II) and a different monomer may be a copolymer including two or more hydrophilic polymers which include the same structural unit and have different molecular weights (for example, the hydrophilic polymers include a copolymer of 2-methoxyetyl acrylate having a number average molecular weight of 16000 and styrene and a copolymer of 2-methoxyetyl acrylate having a number average molecular weight of 80000 and styrene) or may be a copolymer including two or more hydrophilic polymers which include different structural units and have different molecular weights (for example, the hydrophilic polymers include a copolymer of 2-methoxyetyl acrylate having a number average molecular weight of 16000 and styrene and a copolymer of 2-methoxyetyl acrylate having a number average molecular weight of 80000 and vinyl acetate). One type or two or more types of the hydrophilic polymers represented by the formula (II) may be used.

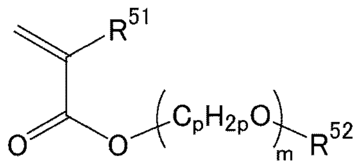

(II)

In the formula, $R^{51}$ represents a hydrogen atom or a methyl group; $R^{52}$ represents an alkyl group; p represents 1 to 8; and m represents 1 to 5.

The compounds (hydrophilic monomers) represented by the formula (II) are preferably compounds represented by the formula (II-1) below. Each of the compounds represented by the formula (II-1) may be used alone or in combinations of two or more.

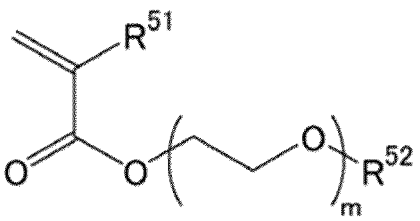

(II-1)

In the formula, $R^{51}$ represents a hydrogen atom or a methyl group; $R^{52}$ represents an alkyl group; and m represents 1 to 5.

In the formulas (I), (I-1), (II), and (II-1), the number of carbon atoms of the alkyl group for $R^{52}$ is preferably 1 to 10, more preferably 1 to 5. $R^{52}$ is particularly preferably a methyl group or an ethyl group. The symbol p is preferably 1 to 5, more preferably 1 to 3. The symbol m is preferably 1 to 3. The symbol n (the number of repeating units) is preferably 15 to 1500, more preferably 40 to 1200.

To form a hydrophilic polymer layer having a low surface roughness and a high flat smoothness by controlling the irregularities of the substrate surface, the hydrophilic polymers represented by the formula (I) are preferred, and the hydrophilic polymers represented by the formula (I-1) are particularly preferred among the above-described hydrophilic polymers.

Non-limiting examples of the molecular weight of the hydrophilic polymers with different molecular weights include number average molecular weight (Mn) and weight average molecular weight (Mw). A blend of hydrophilic polymers with different Mn may suitably be used.

In the case of the blend of hydrophilic polymers with different Mn, the blend preferably includes hydrophilic polymers with a difference in Mn of 30000 or more and more preferably includes hydrophilic polymers with a difference in Mn of 50000 or more to better achieve the advantageous effect.

To better achieve the advantageous effect, the blend of hydrophilic polymers with different molecular weights preferably includes hydrophilic polymers with low molecular weights having a number average molecular weight (Mn) of 5000 or more and 25000 or less. The hydrophilic polymers with low molecular weights each have Mn of preferably 10000 or more, more preferably 14000 or more and preferably 23000 or less.

To better achieve the advantageous effect, the blend of hydrophilic polymers with different molecular weights desirably includes hydrophilic polymers with high molecular weights having a number average molecular weight (Mn) of 35000 or more and 200000 or less. The hydrophilic polymers with high molecular weights each have Mn of preferably 40000 or more, more preferably 50000 or more and preferably 150000 or less, more preferably 130000 or less.

Herein, the number average molecular weight (Mn) and the weight average molecular weight (Mw) can be determined by gel permeation chromatography (GPC) (GPC-8000 series available from Tosoh Corporation, detector: differential refractometer, column: TSKgel SuperMultipore HZ-M available from Tosoh Corporation) and calibrated with polystyrene standards.

The thickness of the hydrophilic polymer blend layer is preferably 30 to 3000 nm, more preferably 30 to 1500 nm, still more preferably 50 to 1000 nm. When the thickness is within the range indicated above, the advantageous effect tends to be better achieved. Further, low adsorption of normal proteins and cells and selective adsorption of specific cells such as cancer cells, stem cells, and T-cells can be expected.

The thickness of the hydrophilic polymer blend layer can be measured by the method described later in EXAMPLES.

The surface of the hydrophilic polymer blend layer at least partially (partially or entirely) has a contact angle with water of preferably 65° or less, more preferably 60° or less. The lower limit is not limited, and a smaller angle is better.

The hydrophilic polymer blend layer may be formed by known methods, such as (1) a method of introducing a hydrophilic polymer solution or dispersion in which two or more hydrophilic polymers with different molecular weights are

dissolved or dispersed in a solvent onto the surface (depressed portions, etc. of the substrate) of the glass substrate, optionally followed by retaining or drying for a predetermined time and (2) a method of applying (spraying) the hydrophilic polymer solution or dispersion onto the surface (depressed portions, etc. of the substrate) of the glass substrate, optionally followed by retaining or drying for a predetermined time. A polymer-coated glass substrate can be produced by forming a hydrophilic polymer blend layer on the surface of the glass substrate by such known methods. Then, the polymer-coated glass substrate may be combined with other components as needed to prepare a device capable of, for example, adsorbing, culturing, and/or analyzing specific cells such as cancer cells, stem cells, and T-cells.

Conventionally known materials or methods are applicable as the solvent, introduction method, application (spraying) method, etc.

The duration of retention/drying in the method (1) or (2) may be appropriately selected depending on the size of the glass substrate, the type of liquid introduced, etc. The duration of the retention is preferably 10 seconds to 10 hours, more preferably 1 minute to 5 hours, still more preferably 5 minutes to 2 hours. The drying is preferably performed at room temperature (about 23°C) to 80°C, more preferably at room temperature to 60°C. Moreover, the drying may be performed under reduced pressure. Further, after a predetermined time of retention, the excess hydrophilic polymer solution or dispersion may be discharged as appropriate before drying.

The solvent may be any solvent that can dissolve hydrophilic polymers and may be appropriately selected depending on the hydrophilic polymers used. For example, the solvent may be water, an organic solvent, or a mixture thereof. Examples of the organic solvent include alcohols such as methanol, ethanol, n-propanol, i-propanol, and methoxypropanol; ketones such as acetone and methyl ethyl ketone; tetrahydrofuran, acetonitrile, ethyl acetate, and toluene. The solvent which is warmed may be used.

The solvent may be used alone or in combinations of two or more.

The concentration of the hydrophilic polymer solution or dispersion is not limited and may be appropriately selected in view of introduction, application, or spraying properties, productivity, etc. The concentration of the hydrophilic polymers in the hydrophilic polymer solution or dispersion (100% by mass) is preferably 0.01 to 10.0% by mass, more preferably 0.10 to 5.0% by mass.

A scaffold protein may be adsorbed onto a surface of the hydrophilic polymer blend layer. The scaffold protein adsorbed onto the surface of the hydrophilic polymer blend layer can facilitate the adsorption of specific cells such as cancer cells, stem cells, and T-cells.

Herein, the term "scaffold protein" refers to a protein that has the function of promoting selective adsorption of the specific cells, such as a protein that has the function of specifically binding to a protein appearing on the surface of the specific cells. The term also refers to a protein that has the function of interacting with (e.g., adsorbing to, binding to, or associating with) the hydrophilic polymers, such as a protein which may adsorb to the hydrophilic polymers to mediate the function of promoting selective adsorption of the specific cells to the surfaces of the hydrophilic polymers.

The scaffold protein desirably has an arginine-glycine-aspartate (RGD) sequence. Suitable examples of the scaffold protein include fibronectin.

The scaffold protein may be adsorbed onto the hydrophilic polymer blend layer by any known method. For example, the scaffold protein may be adsorbed onto the hydrophilic polymer blend layer by bringing the layer into contact with a buffer solution (e.g., phosphate buffered saline (PBS)) of the scaffold protein by a known method, and leaving them at a predetermined temperature for a predetermined time, optionally followed by washing. The temperature and duration may be selected as appropriate, and may be, for example, about 10°C to 60°C and about 0.1 to 24 hours, respectively.

From the standpoint of adsorbing the scaffold protein onto the hydrophilic polymer blend layer, it is suitable to use a solution or dispersion with a concentration of the scaffold protein adjusted to 0.5 to 500 ug/ml, more preferably 1 to 250 ug/ml. When the concentration of the scaffold protein is adjusted within the range indicated above, excellent adsorption of specific cells can be achieved. The concentration of fibronectin is also desirably within the range indicated above.

In the polymer-coated glass substrate, the surface of the hydrophilic polymer blend layer desirably has a low elastic modulus in water or an aqueous solution.

Specific cells such as cancer cells, stem cells, and T-cells are known to be generally softer than normal cells such as blood cells. This is related to the fact that when specific cells such as cancer cells metastasize, they deform their cell shape greatly and move through gaps. Thus, normal cells such as blood cells, which are hard and less deformable, are less likely to be adsorbed to a polymer-coated glass substrate that is coated with hydrophilic polymers having a soft surface. In contrast, specific cells such as cancer cells that have acquired deformability tend to be adsorbed to a polymer-coated glass substrate having a soft surface. An excessively soft polymer-coated glass substrate has a low adsorption capacity. The hydrophilic polymer blend layer on the glass substrate can form a hydrophilic polymer layer having a high flat smoothness and a low elastic modulus (high flexibility), thereby achieving excellent adsorption of specific cells such as cancer cells, stem cells, and T-cells.

To reduce the adsorption of normal cells such as blood cells and selectively adsorb specific cells such as cancer cells, stem cells, and T-cells, the elastic modulus is preferably 1.20 MPa or less, more preferably 0.80 MPa or less, still more preferably 0.50 MPa or less, particularly preferably 0.30 MPa or less. The lower limit is preferably 0.01 MPa or more,

more preferably 0.05 MPa or more.

The elastic modulus in water or an aqueous solution can be controlled by changing the molecular weights of the hydrophilic polymers in the hydrophilic polymer blend layer or the film thickness. Specifically, the elastic modulus tends to increase as the molecular weights of the hydrophilic polymers increase or as the film thickness of the hydrophilic polymer layer increases.

Herein, the term "elastic modulus in water or an aqueous solution" refers to the elastic modulus in water or an aqueous solution measured with an atomic force microscope (AFM), unless otherwise stated.

An atomic force microscope (AFM) is a type of scanning probe microscope which detects the force acting between the atoms on the sample and the atoms on the probe. The probe is attached to the tip of a cantilever (cantilever spring). The force (amount of deflection) acting on the cantilever can be measured while changing the distance between the sample and the probe to obtain a curve (force curve) plotting the relationships between them. Analysis of the force curve can determine the elastic modulus (hardness) of the surface of the sample at the nano scale. The method for determining the elastic modulus of the surface of the sample by the force curve analysis is known to those skilled in the art. The elastic modulus can be determined by such a known method.

For example, the elastic modulus may be calculated from the force curve by fitting the force curve according to the Johnson-Kendall-Roberts (JKR) theory to calculate the elastic modulus. According to the JKR theory, the force F applied to the cantilever and the amount of deformation $\delta$ of the sample are expressed by the following equations (1) and (2) with w representing the adhesion energy.

$$F = \frac{a^3}{R} - \sqrt{6\pi K w a^3} \qquad (1)$$

$$\delta = \frac{a^2}{3R} + \frac{2F}{3aK} \qquad (2)$$

In the equations, a denotes the radius of a contact line between the probe and the sample, R denotes the radius of curvature of the probe tip, and K denotes the elastic coefficient.

The elastic modulus can be determined from the F-$\delta$ curve obtained by the force curve analysis and the fitting using the equations (1) and (2).

Here, the elastic modulus in water or an aqueous solution specifically refers to the value measured as follows.

The elastic modulus in water or an aqueous solution can be measured with an AFM by dropping water or an aqueous solution on the surface of the sample to form a droplet (convex meniscus).

Suitable examples of the aqueous solution include phosphate buffered saline (PBS).

Then, the elastic modulus of the sample (the surface of the hydrophilic polymer blend layer) may be determined, for example, by scanning the surface of the sample within a predetermined range to acquire force curves at multiple points within the predetermined range, determining the elastic moduli from the force curves, and calculating the average of the elastic moduli.

The polymer-coated glass substrate includes a hydrophilic polymer layer that includes a blend of two or more hydrophilic polymers with different molecular weights on the surface of the glass substrate, whereby the irregularities of the substrate surface can be controlled. Thus, a hydrophilic polymer layer having a low surface roughness and a high flat smoothness can be formed. The hydrophilic polymer layer enables excellent adsorption of specific cells such as cancer cells, stem cells, and T-cells. The surface of the hydrophilic polymer blend layer has a low elastic modulus and can exhibit much better adsorption of specific cells. Therefore, the number of specific cells in sampled blood or biological fluid can be determined by counting the number of adsorbed specific cells, and the determined number can be used to evaluate the cancer-treating effect, etc. Moreover, the adsorbed specific cells may be cultured and then used to determine the effects of drugs such as anticancer drugs or to screen anticancer drugs.

EXAMPLES

The present invention is specifically described below based on examples, but the present invention is not limited to the examples.

<Production of hydrophilic polymer>

(Production of polymer 1)

Using a solution of azobisisobutyronitrile (AIBN) in toluene (1.25 mg/ml), 2-methoxyethyl acrylate (25 wt% in the toluene solution) was thermally polymerized at 60°C for seven hours to produce poly(2-methoxyethyl acrylate) (PMEA, Mn: 16000).

(Production of polymer 2)

Using a solution of azobisisobutyronitrile (AIBN) in methanol (1.25 mg/ml), 2-methoxyethyl acrylate (50 wt% in the methanol solution) was thermally polymerized at 60°C for seven hours to produce poly(2-methoxyethyl acrylate) (PMEA, Mn: 80000).

<Production of polymer-coated glass substrate>

(Example 1)

A 0.38 wt% solution of the PMEA (Mn: 16000) produced in the production of polymer 1 in methanol (the methanol solution was warmed to 40°C) and a 0.08 wt% solution of the PMEA (Mn: 80000) produced in the production of polymer 2 in methanol (the methanol solution was warmed to 40°C) were mixed at a mass ratio of 90:80. The resulting solution (the methanol solution was warmed to 40°C) in an amount of 85 μl was introduced into one well of a slide chamber (two-well type) (uncoated, available from Matsunami Glass Ind., Ltd., bottom face: soda lime glass, contact angle in water: 109.9°, average thickness: 1.35 mm), immediately followed by vacuum drying in an oven at 50°C for 20 minutes. Thus, a polymer-coated glass substrate was produced.

(Example 2)

The PMEA (Mn: 16000) produced in the production of polymer 1 was dissolved in methanol to a concentration of 0.33 wt% (the methanol solution was warmed to 40°C). Then, the PMEA (Mn: 80000) produced in the production of polymer 2 was dissolved in the methanol solution to a concentration of 0.04 wt% (the methanol solution was warmed to 40°C). The resulting solution (the methanol solution was warmed to 40°C) in an amount of 85 μl was introduced into one well of a slide chamber (two-well type) (uncoated, available from Matsunami Glass Ind., Ltd., bottom face: soda lime glass, contact angle in water: 109.9°, average thickness: 1.35 mm), immediately followed by vacuum drying in an oven at 50°C for 20 minutes. Thus, a polymer-coated glass substrate was produced.

(Example 3)

The PMEA (Mn: 16000) produced in the production of polymer 1 was dissolved in methanol to a concentration of 0.48 wt% (the methanol solution was warmed to 40°C). Then, the PMEA (Mn: 80000) produced in the production of polymer 2 was dissolved in the methanol solution to a concentration of 0.13 wt% (the methanol solution was warmed to 40°C). The resulting solution (the methanol solution was warmed to 40°C) in an amount of 85 μl was introduced into one well of a slide chamber (two-well type) (uncoated, available from Matsunami Glass Ind., Ltd., bottom face: soda lime glass, contact angle in water: 109.9°, average thickness: 1.35 mm), immediately followed by vacuum drying in an oven at 50°C for 20 minutes. Thus, a polymer-coated glass substrate was produced.

(Comparative Example 1)

The PMEA (Mn: 80000) produced in the production of polymer 2 was dissolved in methanol to a concentration of 0.25 wt% (the methanol solution was warmed to 40°C). The resulting solution (the methanol solution was warmed to 40°C) in an amount of 85 μl was introduced into one well of a slide chamber (two-well type) (uncoated, available from Matsunami Glass Ind., Ltd., bottom face: soda lime glass, contact angle in water: 109.9°, average thickness: 1.35 mm), immediately followed by vacuum drying in an oven at 50°C for 20 minutes. Thus, a polymer-coated glass substrate was produced.

The polymer-coated glass substrates produced in the examples and the comparative example were analyzed for occurrence of white turbidity of the polymer layer, the elastic modulus of the surface in phosphate buffered saline (PBS) measured with AFM, and the thickness of the hydrophilic polymer layer by the methods below. Table 1 shows the results.

[Thickness of hydrophilic polymer layer]

A cross section of the hydrophilic polymer layer was measured (photographed) using TEM (JEOL, JEM-2800) at

an acceleration voltage of 200 kV.

[Occurrence of white turbidity of polymer layer]

After two hours from the coating, the surface was visually observed.
Occurrence of white turbidity indicates that the surface has a large surface roughness. The surface with less white turbidity has a higher flat smoothness.

[Elastic modulus of surface]

Phosphate buffered saline was dropped on the surface of the hydrophilic polymer layer of the polymer-coated glass substrate to form a droplet (convex meniscus). The elastic modulus of the surface was measured by the following method using the device (AFM) described below. The determined elastic modulus was defined as the elastic modulus measured in water or an aqueous solution. In the measurement of the elastic modulus, the obtained force curve was analyzed based on the JKR contact theory to determine the elastic modulus.

<Method for measuring elastic modulus>

Device (AFM): MFP-3D-SA available from Oxford Instruments
Measurement mode: AFM force curve mapping
Cantilever: material Si, radius of curvature of tip R = 150 nm, spring constant 0.67 N/m
Measurement range: scan of 20 um × 20 um, calculation of elastic modulus by 2-point JKR method
Scanning rate: 1 Hz
Measurement environment: in PBS
Measurement temperature: 23°C

A lower elastic modulus indicates better adsorption of specific cells such as cancer cells.

[Table 1]

| | Example 1 | Example 2 | Example 3 | Comparative Example 1 |
|---|---|---|---|---|
| Thickness of hydrophilic polymer layer (nm) | 198 | 195 | 192 | 200 |
| White turbidity of polymer layer | Not occurred | Not occurred | Not occurred | Not occurred |
| Elastic modulus of surface (MPa) | 0.147 | 0.167 | 0.182 | 1.256 |

Exemplary embodiments of the present invention include the following.
Embodiment 1. A polymer-coated glass substrate including

a glass substrate and
a hydrophilic polymer layer including a blend of hydrophilic polymers with different molecular weights, the hydrophilic polymer layer being on a surface of the glass substrate.

Embodiment 2. The polymer-coated glass substrate according to Embodiment 1,
wherein the hydrophilic polymers include hydrophilic polymers with a difference in number average molecular weight of 30000 or more.
Embodiment 3. The polymer-coated glass substrate according to Embodiment 1,
wherein the hydrophilic polymers include hydrophilic polymers with a difference in number average molecular weight of 50000 or more.
Embodiment 4. The polymer-coated glass substrate according to any combination with any one of Embodiments 1 to 3,
wherein the hydrophilic polymers include hydrophilic polymers with different number average molecular weights represented by the following formula (I):

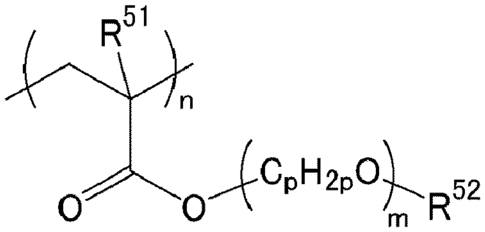

(I)

wherein $R^{51}$ represents a hydrogen atom or a methyl group; $R^{52}$ represents an alkyl group; p represents 1 to 8; m represents 1 to 5; and n represents the number of repetitions.

Embodiment 5. The polymer-coated glass substrate according to any combination with any one of Embodiments 1 to 3,
wherein the hydrophilic polymers include a copolymer of hydrophilic monomers with different number average molecular weights represented by the formula (II) below and a different monomer:

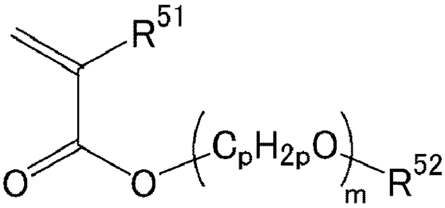

(II)

wherein $R^{51}$ represents a hydrogen atom or a methyl group; $R^{52}$ represents an alkyl group; p represents 1 to 8; and m represents 1 to 5.

Embodiment 6. The polymer-coated glass substrate according to any combination with any one of Embodiments 1 to 5,
wherein the hydrophilic polymer layer has a thickness of 30 to 3000 nm.

Embodiment 7. The polymer-coated glass substrate according to any combination with any one of Embodiments 1 to 6,
wherein a scaffold protein is adsorbed onto a surface of the hydrophilic polymer layer.

Embodiment 8. The polymer-coated glass substrate according to Embodiment 7,
wherein the scaffold protein is fibronectin.

**Claims**

1. A polymer-coated glass substrate comprising

   a glass substrate and
   a hydrophilic polymer layer comprising a blend of hydrophilic polymers with different molecular weights, the hydrophilic polymer layer being on a surface of the glass substrate.

2. The polymer-coated glass substrate according to claim 1,
   wherein the hydrophilic polymers include hydrophilic polymers with a difference in number average molecular weight of 30000 or more.

3. The polymer-coated glass substrate according to claim 1,
   wherein the hydrophilic polymers include hydrophilic polymers with a difference in number average molecular weight of 50000 or more.

4. The polymer-coated glass substrate according to any one of claims 1 to 3,
   wherein the hydrophilic polymers include hydrophilic polymers with different number average molecular weights represented by the following formula (I):

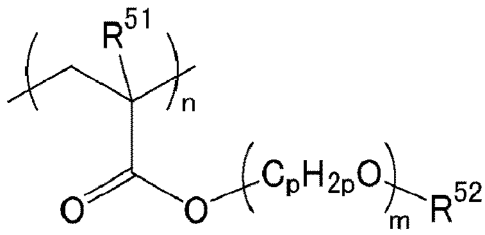

(I)

wherein $R^{51}$ represents a hydrogen atom or a methyl group; $R^{52}$ represents an alkyl group; p represents 1 to 8; m represents 1 to 5; and n represents the number of repetitions.

**5.** The polymer-coated glass substrate according to any one of claims 1 to 3,
wherein the hydrophilic polymers include a copolymer of hydrophilic monomers with different number average molecular weights represented by the formula (II) below and a different monomer:

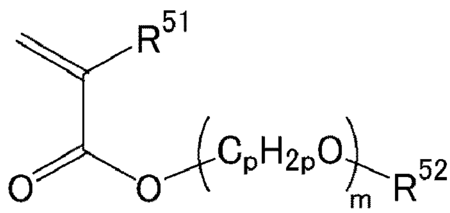

(II)

wherein $R^{51}$ represents a hydrogen atom or a methyl group; $R^{52}$ represents an alkyl group; p represents 1 to 8; and m represents 1 to 5.

**6.** The polymer-coated glass substrate according to any one of claims 1 to 5,
wherein the hydrophilic polymer layer has a thickness of 30 to 3000 nm.

**7.** The polymer-coated glass substrate according to any one of claims 1 to 6,
wherein a scaffold protein is adsorbed onto a surface of the hydrophilic polymer layer.

**8.** The polymer-coated glass substrate according to claim 7,
wherein the scaffold protein is fibronectin.

Europäisches Patentamt
European Patent Office
Office européen des brevets

# EUROPEAN SEARCH REPORT

Application Number
EP 24 21 7147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 915 957 A1 (SUMITOMO RUBBER IND [JP]) 1 December 2021 (2021-12-01) * paragraph [0070] - paragraph [0071]; examples 1,3 * | 1-8 | INV. C03C17/00 C03C17/32 |
| X | EP 3 670 463 A1 (SUMITOMO RUBBER IND [JP]) 24 June 2020 (2020-06-24) * examples * | 1,4-8 | |
| X | US 2015/118501 A1 (LU YONGSHANG [US] ET AL) 30 April 2015 (2015-04-30) * paragraph [0031]; examples 4,5 * | 1-3 | |

TECHNICAL FIELDS SEARCHED (IPC)
C03C

The present search report has been drawn up for all claims

2

| Place of search | Date of completion of the search | Examiner |
|---|---|---|
| Munich | 28 April 2025 | Pollio, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 21 7147

This annex lists the patent family members relating to the patent documents cited in the above-mentioned European search report. The members are as contained in the European Patent Office EDP file on
The European Patent Office is in no way liable for these particulars which are merely given for the purpose of information.

28-04-2025

| Patent document cited in search report | | Publication date | Patent family member(s) | | | Publication date |
|---|---|---|---|---|---|---|
| EP 3915957 | A1 | 01-12-2021 | CN | 113735457 | A | 03-12-2021 |
| | | | EP | 3915957 | A1 | 01-12-2021 |
| | | | JP | 7639275 | B2 | 05-03-2025 |
| | | | JP | 2021187907 | A | 13-12-2021 |
| | | | US | 2021372895 | A1 | 02-12-2021 |
| EP 3670463 | A1 | 24-06-2020 | EP | 3670463 | A1 | 24-06-2020 |
| | | | JP | 7415320 | B2 | 17-01-2024 |
| | | | JP | 2020100062 | A | 02-07-2020 |
| US 2015118501 | A1 | 30-04-2015 | CN | 104080861 | A | 01-10-2014 |
| | | | EP | 2791257 | A1 | 22-10-2014 |
| | | | JP | 5855277 | B2 | 09-02-2016 |
| | | | JP | 2015505889 | A | 26-02-2015 |
| | | | TW | 201323535 | A | 16-06-2013 |
| | | | US | 2015118501 | A1 | 30-04-2015 |
| | | | WO | 2013089927 | A1 | 20-06-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005523981 T